# EUROPEAN PATENT SPECIFICATION

(11) **EP 4 418 989 B1**
(45) Date of publication and mention of the grant of the patent: **09.12.2026**
(21) Application number: 22882101.3
(22) Date of filing: 18.10.2022
(51) Int. Cl.: A61B 5/00, A61B 5/055, A61B 8/00, A61B 8/08, G01B 11/16, G01B 9/02091, G01L 1/24

(54) **AN ATTACHMENT FOR AN ELASTOGRAPHY AND/OR IMAGING DEVICE**
AUFSATZ FÜR EINE ELASTOGRAPHIE- UND/ODER BILDGEBUNGSVORRICHTUNG
ACCESSOIRE POUR UN DISPOSITIF D'ÉLASTOGRAPHIE ET/OU D'IMAGERIE

(30) Priority: 19.10.2021 AU 2021903355
(43) Date of publication of application: 28.08.2024
(73) Proprietor: OncoRes Medical Pty Ltd, Nedlands, Western Australia 6009 (AU)
(72) Inventor: KENNEDY, Brendan, Walliston, Western Australia, 6076 (AU); WIJESINGHE, Philip, St Andrews, KY168HF (GB); ANSTIE, James, Mount Lawley, Western Australia, 6050 (AU); FIRTH, Daniel, Highgate, Western Australia, 6003 (AU); FREWER, Luke, Mount Lawley, Western Australia, 6050 (AU)
(74) Representative: Herrmann, Jochen
(86) International application number: PCT/AU2022/051251
(87) International publication number: WO 2023/064982

(56) References cited:
- WO-A1-2016/119011
- WO-A1-2017/185145
- WO-A1-2019/071295
- WO-A1-2020/082133
- WO-A1-2020/082133
- US-A1- 2009 116 032
- US-A1- 2012 265 061
- US-A1- 2018 042 480
- US-B1- 6 261 231
- KENNEDY BRENDAN FRANCIS; KENNEDY KELSEY MARIE; SAMPSON DAVID DOUGLAS: "A Review of Optical Coherence Elastography: Fundamentals, Techniques and Prospects", IEEE JOURNAL OF SELECTED TOPICS IN QUANTUM ELECTRONICS, IEEE, USA, vol. 20, no. 2, 1 March 2014 (2014-03-01), USA, pages 1 - 17, XP011536873, ISSN: 1077-260X, DOI: 10.1109/JSTQE.2013.2291445
- LI J., HAMMER S.J., SHU W.M., MAIER R.R.J., HAND D.P., REUBEN R.L., MACPHERSON W.N.: "An optical fibre dynamic instrumented palpation sensor for the characterisation of biological tissue", SENSORS AND ACTUATORS A: PHYSICAL, ELSEVIER BV, NL, vol. 225, 1 April 2015 (2015-04-01), NL , pages 53 - 60, XP093062934, ISSN: 0924-4247, DOI: 10.1016/j.sna.2015.01.036

## Description

### Field of the Invention

The present invention relates to an attachment for an elastography device and/or imaging device, and more specifically, although not exclusively, to a removable attachment for an optical elastography device.

### Background of the Invention

Elastography techniques based on optical imaging, ultrasound imaging and magnetic resonance imaging (MRI) are commonly used to characterise deformation of a sample material, such as a biological tissue, and evaluate stiffness and other mechanical properties of the sample.

In recent years, there has been development in elastography techniques such as in optical coherence tomography (OCT) - based elastography. The present applicant has developed an optical elastography technique that uses a compliant sensing layer compressed against a surface of a sample material. This technique is disclosed in PCT international patent application no. PCT/AU2016/000019.

The present applicant has further developed an optical elastography device, which is digital camera-based and may be handheld. This device and a method for evaluating a mechanical property, such as elasticity, of a sample material using the optical elastography device, are disclosed in PCT international patent application PCT/AU2019/051171.

US 6 261 231 B1 discloses an attachment suitable for an elastography and/or imaging device, the device having a transmission portion for transmission of acoustic waves towards a sample material, the attachment comprising:
a. a securing portion for securing the attachment to the device;
b. a sensing portion coupled to the securing portion, the sensing portion being adapted to receive a deformable sensing layer that is at least partially transmissive for the acoustic waves;
c. a ring-like portion coupled to the securing portion;
d. wherein the attachment is arranged such that, when attached to the device and the sensing layer is received at the sensing portion, the acoustic waves are transmitted through the sensing layer towards the sample material, and when a load is applied to the sample material through the sensing layer, the sensing layer deforms.

The present invention provides further improvement.

### Summary of the Invention

In accordance with a first aspect of the present invention, there is provided an attachment for an elastography and/or imaging device according to claim 1.

Further aspects are defined in the dependent claims.

### Brief Description of the Drawings

Embodiments of the present invention will now be described, by way of example only, with reference to the accompanying figures, in which:
Figure 1 (a) is a schematic perspective representation of an attachment for an elastography and/or imaging device in accordance with an embodiment with the attachment being secured to an end portion of a probe of the device;
Figure 1 (b) is a schematic perspective representation of the attachment of Figure 1 (a);
Figure 2 is a schematic perspective representation of an attachment for an elastography device in accordance with another embodiment with the attachment being secured to a probe of the device;
Figure 3 is a perspective view of the attachment of Figure 2, when not attached to the probe of the device;
Figure 4 is a photograph of the attachment of Figures 2 and 3; and
Figure 5 is a schematic representation of an optical elastography device in accordance with an embodiment.

### Detailed Description of Specific Embodiments

Ultrasound elastography, MRI-based elastography and optical coherence elastography techniques can be used to map the mechanical properties of biological tissue, such as stiffness (elasticity). As the mechanical properties of biological tissue may be affected by diseases such as cancer, one of the applications of these techniques relates to the identification of cancerous tissue, which is usually "stiffer" than surrounding soft tissue.

The present applicant has previously developed an optical elastography technique in which a compressive load is applied to a sample material through a deformable sensing layer positioned between a probe and the sample material. The sensing layer comprises a transparent silicone material, which is incompressible and, therefore, deforms under the application of the compressive load by expanding in a plane transversal to the applied load to preserve its volume. The thickness of the sensing layer thus changes in response to the local stiffness of the underlying sample material and using, for example optical, coherence tomography (OCT), the change in thickness of the sensing layer induced by the compressive load can be measured to provide a measure for the local stress of the underlying sample material. Using OCT, strain in the sample can also be determined, which together with the determined stress in the sample can provide the stiffness of the sample.

Embodiments of the present invention provide an attachment for an elastography and/or imaging device that is arranged such that a deformable sensing layer having an incompressible volume can be used without compromising the change in thickness of the sensing layer (for example by obstructing the lateral expansion of the layer) as a function of a change in "stiffness' of the underlying sample material.

In a specific embodiment, the attachment is disposable and is adapted for removably securing to the elastography and/or imaging device.

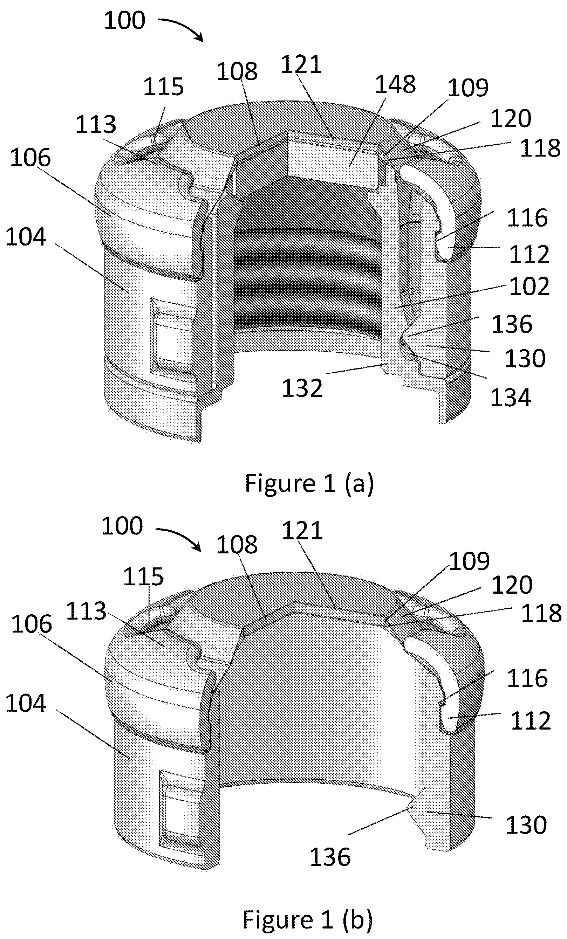

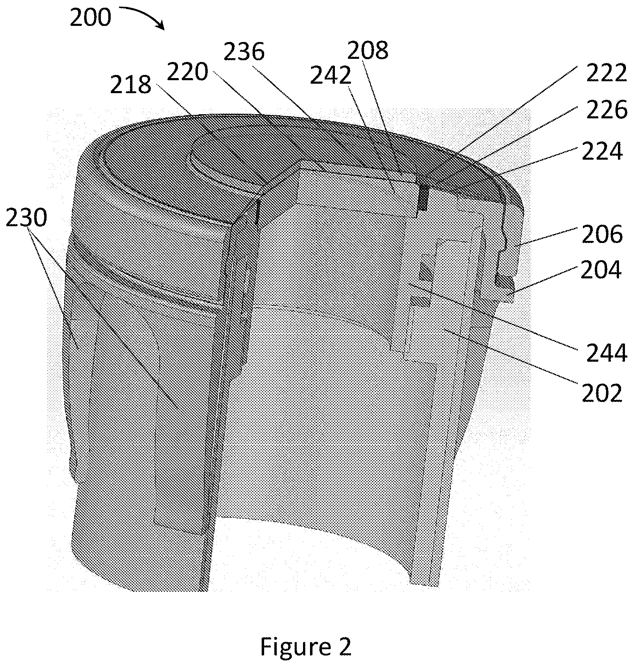

With reference to Figures 1 and 2, a specific embodiment of the attachment will be described. In this embodiment the attachment is suitable for an optical elastography device and is adapted to be secured to an optical probe of the device. However, it will be understood that embodiments of the present invention are not limited to applications in optical elastography. For example, the device may alternatively be an ultrasonic device or an MRI device and may or may not be an imaging device.

Figure 1 (a) is a perspective view of an attachment 100 secured to an optical probe 102 of an optical elastography device and Figure 1(b) shows the attachment 100 in isolation. The attachment 100 comprises a securing portion 104 for securing the attachment 100 on the optical probe 102. The optical probe 102 has a transmission portion (not shown) for transmission of electromagnetic radiation towards the sample material (also not shown). The attachment 100 further comprises a ring-like portion 106, which is coupled to the securing portion 104 and is adapted to receive a deformable sensing layer 108 that is at least partially transmissive for the electromagnetic radiation. The attachment 100 is arranged such that, when attached to the probe 102 and the sensing layer 108 is received (as shown in Figures 1(a) and 1(b)), the electromagnetic radiation is transmitted through the sensing layer 108 towards the sample material, and when an axial load is applied through the sensing layer 108, the sensing layer 108 can expand laterally relative to a longitudinal axis of the probe 102.

A person skilled in the art will appreciate that in an alternative embodiment in which the device is an ultrasonic device, the probe will have a transmission portion for transmission of acoustic or ultrasonic waves and the attachment will be adapted to receive a sensing layer that is at least partially transmissive for the acoustic or ultrasonic waves.

The attachment 100 with the sensing layer 108 has a U-shaped cross-sectional shape and is generally cylindrical and components of the attachment 100 are formed from a flexible polymeric material. As will now be described in more detail, the ring-like portion 106 is in this example coupled to the securing portion 104 using an annular compression. An inner surface of the ring-like portion 106 has a projection 112 and an outer surface of the securing portion 104 has a corresponding recess 116 which is arranged to engage with the projection 112.

In the present embodiment, the attachment 100 comprises the sensing layer 108, which is coupled to the ring-like portion 106 using layers 118, 120. The sensing layer 108 is sandwiched between the layers 118, 120, which hold the sensing layer 108 at the ring-like portion 106 and are formed from a flexible material. The attachment 100 is arranged such that, when attached to the probe 102 and the sensing layer 108 is received and coupled to the ring-like portion 106, a sensing surface 121 of the sensing layer 108 is in indirect contact with the sample material (not shown).

The layers 118, 120 are secured between the ring-like portion 106 and the securing portion 104 and an outer dimension of the sensing layer 108 is chosen such that a cavity 109 is defined between the outer portions of the layers 118, 120 at an edge-portion of the sensing layer 108. The sensing layer 108 is located by the layers 118, 120 so as to enable a lateral expansion of the sensing layer 108 into the cavity 109 relative to a longitudinal axis of the probe 102 when an axial load is applied through the sensing layer 108.

A lubrication material is also provided between the layers 118, 120 and the sensing layer 108. The lubrication material is selected to reduce friction between the sensing layer 108 and the layers 118, 120. The attachment 100 is arranged such that, when the axial load is applied through the sensing layer 108, the lubrication material is allowed to flow into the cavity 109 into which the material of the sensing layer 108 can also laterally expand. The lubrication material may be any suitable material reduce friction between the sensing layer 108 and the layers 118, 120 and may be for example be a silicone oil, a vegetable oil or a synthetic liquid such as hydrogenated polyolefins, esters, or fluorocarbons. By reducing the friction between the sensing layer 108 and the layers 118, 120 the change in thickness more accurately corresponds to change in 'stiffness' of the underlying sample material. Consequently, the resolution of the optical elastography technique can be improved.

The ring-like portion 106 also has an end-portion which has an undulated edge formed by projections 113 projecting inwardly and recesses 115. The end-portion is arranged such that a portion of the sensing layer 108 and/or the lubrication material can penetrate into or through the recesses 115 between the projections 113 when the axial load is applied to the sample material through the sensing layer 108. The projections 113 and also further portions of the attachment 100 may comprise a radiopaque material or coating.

The securing portion 104 has a side portion 130 that is arranged for engaging with a side portion 132 of the probe 102 using a twist-lock or Luer-lock mechanism. An inner surface of the side portion 130 comprises a projection 136 and an outer surface portion of the probe 102 has a recess 134. Further, the probe 102 has a keyway (not shown) along which a projection (or "key") 136 of the side portion 130 can be guided in a direction along an axis of the attachment 100. A twisting movement of the attachment 100 relative to the probe 102 then engages the projection 136 with the recess 134 to secure the attachment 100 onto the probe 102.

The probe 102 comprises an imaging window 148. In a variation of the described embodiment the imaging window may also form a part of the attachment 100 and may be positioned at the layer 120. An adhesive material may be used to hold the imaging window at the layer 118. Electromagnetic radiation is directed from the probe 102 through the imaging window 148 and subsequently through the sensing layer 108.

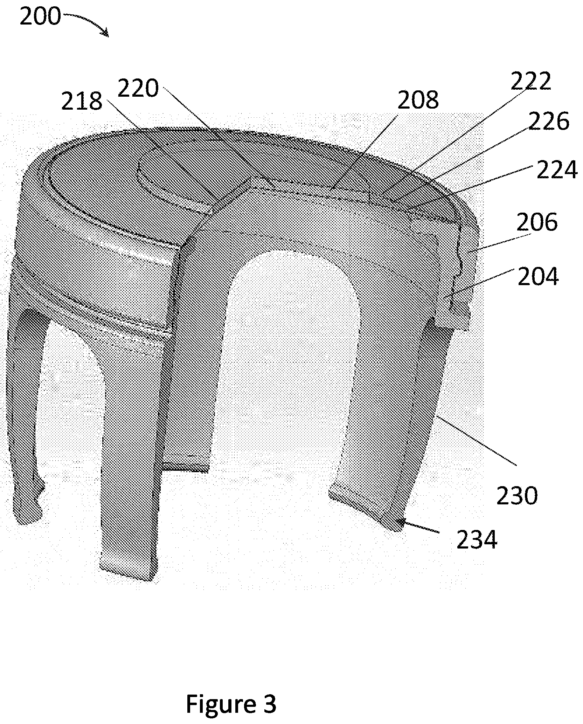

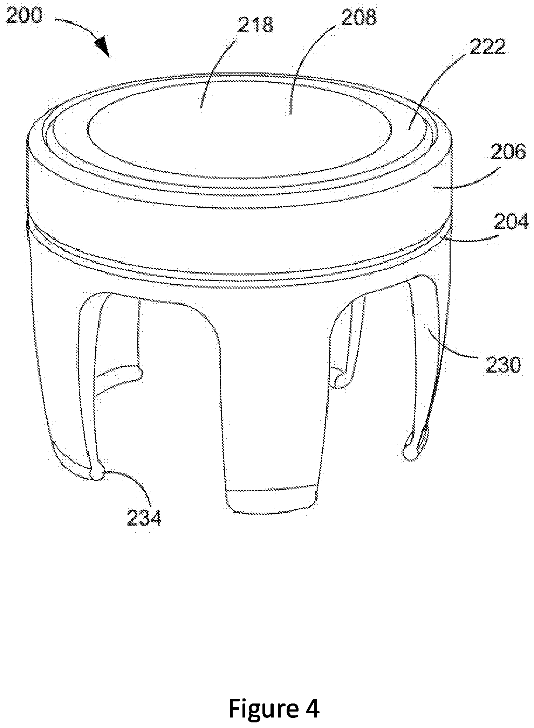

Figures 2, 3 and 4 illustrate an attachment 200 in accordance with another embodiment of the present invention. The attachment 200 is adapted for securing to an optical probe 202. The attachment 200 comprises a securing portion 204 for securing the attachment 200 to the probe 202 of a device, such as an optical elastography device. The probe 202 is in this embodiment an optical probe and has a transmission portion (not shown) for transmission of electromagnetic radiation towards a sample material (not shown). The attachment 200 further comprises a ring-like portion 206, which is coupled to the securing portion 204 by means of an arrangement similar to the arrangement described with reference to Figures 1 (a) and 1 (b). The ring-like portion 206 is adapted to receive a sensing layer 208 that is at least partially transmissive for the electromagnetic radiation. The attachment 200 is arranged such that, when attached to the optical probe 202 and the sensing layer 208 is received at the ring-like portion 206 (as shown in Figure 2), the electromagnetic radiation is transmitted through the sensing layer 208 to the sample material, and when an axial load is applied through the sensing layer 208, the sensing layer 208 can expand laterally relative to a longitudinal axis of the probe 202.

The attachment 200 comprises the sensing layer 208, which is coupled to the ring-like portion 206 using layers 218, 220 of a flexible material as was described for attachment 100 above with reference to Figures 1 (a) and 1 (b). The attachment 200 has a U-shaped cross-sectional shape and is generally cylindrical. The ring-like portion 206 is coupled to the securing portion 204 using a snap-fit mechanism. The sensing layer 208 is sandwiched between the layers 218, 220, which hold the sensing layer 208 at the ring-like portion 206. The sensing layer 208 has dimensions selected such that a lateral expansion of the sensing layer 208 between the layers 218, 220 is possible. The layers 218, 220 are secured between the surfaces of the ring-like portion 206 and the securing portion 204 in a manner such that a cavity 226 is defined between the of layers 218, 220. A lubrication material is provided at the periphery of the sensing layer 208. The lubrication material is arranged to reduce friction between the sensing layer 208 and the layers 218, 220. As for the attachment 100, the lubrication material may be any suitable material that reduces friction between the sensing layer 208 and the layers 218, 220 and may for example be a silicone oil, a vegetable oil or a synthetic liquid such as hydrogenated polyolefins, esters or fluorocarbons.

The securing portion 204 has in this embodiment 5 side portions or prongs 230 that are arranged for engaging with a side portion 232 of the probe 202 in a friction-fit. The attachment 200 has dimensions suitable for being secured to the probe 202 and each side portion 230 has dimensions and a shape such that each side portion 230 can maintain in firm contact with the probe 202 when the attachment 200 is positioned on the probe 202.

The attachment 200 is thus arranged such that, when attached to the probe 202 (as shown in Figure 2), a sensing surface 236 of the sensing layer 208 is adapted for positioning in indirect contact with a surface area of the sample material.

In one embodiment, the attachment 200 comprises an imaging window 242 at the layer 220 whereby, when the attachment 200 is attached to the end portion of the probe 202, the imaging window 242 is positioned between the end of the probe 202 and the sensing layer 208. This arrangement is such that the electromagnetic radiation is directed from the probe 202 through the imaging window 242 and subsequently through the sensing layer 208 to the sample material. In the embodiment shown in Figures 2 to 4 a window mount 244 is further provided to accommodate the imaging window 242 to the shape of the probe 202.

The attachments 100, 200 in accordance with either one of the described embodiments comprises a protective sheath (not shown) which is attached to the securing portion 104, 204 or the ring-like portion 106, 206 in any suitable manner such that when the attachment 100, 200 is secured to the probe 102, 202, the protective sheath extends along the securing portion 104, 204 of the attachment 100, 200 and is clamped between elements of the securing portion 104, 204. For example, an end portion of the protective sheath may be secured between the inner surfaces of the ring-like portion 106, 206 and the outer surfaces of the securing portion 104, 204. The protective sheath may also extend along a portion of an exposed area of the device when the attachment 100, 200 is attached to the device. Alternatively, the attachment 100, 200, may comprise a further sheath (not shown) which is also clamped between elements of the securing portion 104, 204 but extends to cover an exposed area of the device 100, 200 when the attachment 100 is attached to the device.

When the attachment 100, 200 is secured to the probe 102, 202 and the optical elastography device is used, a sensing surface 121, 236 of the sensing layer 108, 208 is positioned in indirect contact with a surface area of a sample material (not shown) through the layer 118, 218. The layers 118, 120, 218, 220 assist in preventing contamination of the sample material and the probe, and in ensuring sterile conditions during use.

The sample material may be a biological tissue or a biological material. Alternatively, the sample material may comprise another elastic or material, such as a polymeric material that may have a non-uniform hardness or flexibility.

The sensing layers 108, 208 may comprise a silicone material or another suitable material.

It will be understood that other materials that are and are at least partially transmissive for the electromagnetic radiation are further envisaged for the sensing layer. Further, embodiments are envisaged in which the sensing layer is receiving in a manner such that, when the attachment is secured to the probe of the device and the device is used, a sensing surface of the sensing layer is positioned in direct contact with a surface area of a sample material.

In an alternative embodiment the attachment uses a deformable sensing layer that has a deformation dependent optical property. In this embodiment the change in thickness of the sensing layer upon application of the compressive load may not have to be detected, but the change in the optical property (such as a change in colour or transmissivity of the layer; the change in the optical property may for example be detected using stereoscopic vison or UV fluorescence technologies as disclosed in the applicant's co-pending PCT international application PCT/AU2019/051171) is a measure for the change in thickness (and the stiffness of the underlying sample material).

Further, in an embodiment in which the probe is an acoustic probe, the sensing layer comprises a deformable material that is at least partially transmissive for acoustic waves.

All parts of the attachment 100, 200 are in this embodiment formed from a bio-compatible material. Alternatively, it is envisaged that at least outer surface portions of the attachment 100, 200 be formed from a bio-compatible material. Further, in one embodiment, all parts of the attachment 100, 200 are disposable.

It will be understood that other embodiments are envisaged for the attachment wherein the sensing portion may be coupled to the securing portion in any other suitable manner and wherein the sensing layer may be received at the sensing portion in any other suitable manner.

The attachment 100, 200 is in this embodiment attached to an optical probe of a hand-held optical elastography device for evaluating a mechanical property of a sample material, such as is disclosed in the present Applicant's PCT international patent application PCT/AU2019/051171. However, a skilled addressee will understand that the attachment in accordance with embodiments of the present invention may also be attached to an optical probe of a hand-held optical imaging device that has no elastography capabilities and or a probe of an ultrasonic device or an MRI device.

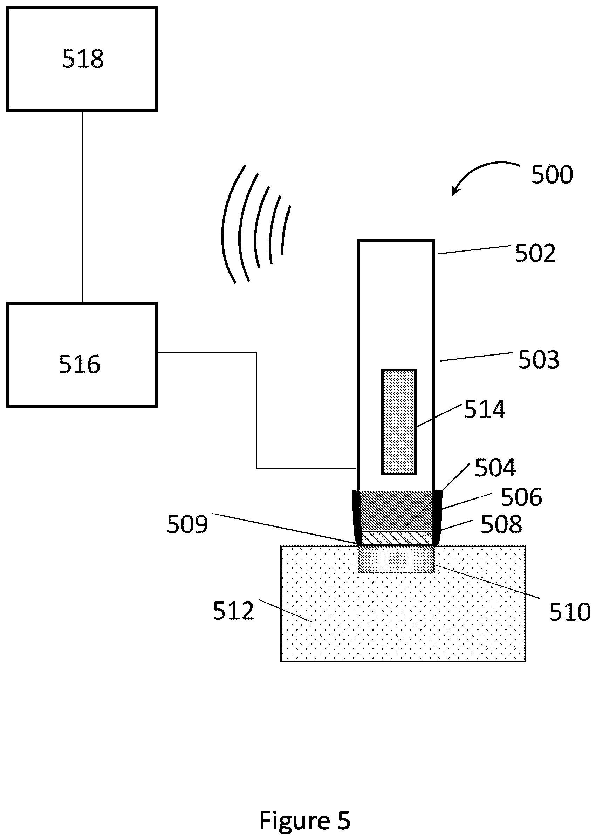

One embodiment of the present invention uses an elastography system comprising an elastography device such as mentioned above, an attachment such as attachments 100 or 200 attached to the elastography device, and a deformable sensing layer that is coupled to the attachment at the ring-like portions 106, 206. Figure 5 illustrates an optical elastography system 500 that comprises a hand-held optical elastography device 502 provided in the shape of a pen. The hand-held optical elastography device 502 comprises an elongated hand-held optical probe 503 having a transmission portion 504 at its end portion for transmission of electromagnetic radiation towards a sample material 512, an attachment 506 that is secured to the end portion 504 of the elongated optical probe 503 of the elastography device 502 and that is similar to either one of the schematically represented attachments 100, 200. The optical elastography system 500 thus also comprises a sensing layer 508 that is coupled to the attachment 506.The hand-held optical elastography device 502 is positioned with a sensing surface 509 of the sensing layer 508 in indirect contact (through the flexible layer, such as layer 118 or 218) at a surface area 510 of the sample material 512, such as a biological tissue. In this embodiment, the sensing layer 508 has a predetermined deformation-dependent optical property and comprises a material that changes colour or transmissivity upon application of an axial load. The hand-held probe 502 is camera based and is equipped with a light detector 514 that is positioned such that light transmitted through the sensing layer 508 from the sample area 510 of the sample material 512, in response to emitting and transmitting light through the sensing layer 508 towards the surface area 510, can be detected. In this regard, it is envisaged that a light source (not shown) is provided within the optical probe 502 for directing light through the sensing layer 508. In the present illustrated example, the optical probe 502 comprises the light detector 514, which is provided in the form of a camera, such as a digital charge coupled device (CCD) camera. The optical elastography system 500 is arranged such that the electromagnetic radiation is transmitted through the sensing layer 508 towards the sample material 512 and when an axial load is applied through the sensing layer 508, the sensing layer 508 can expand laterally relative to a longitudinal axis of the optical probe 503 and elastography device 502. Because of the predetermined deformation-dependent optical property of the sensing layer 508, deformation of the sensing layer 508 influences the light within the sensing layer 508 such that the light detected by the camera 514 is a measure for the change in optical property of the sensing layer and consequently for the mechanical property of the sample material 512 at the surface area 510. In a variation of the described embodiment the camera 514 may be replaced by a suitable stereoscopic vison set up as disclosed in the applicants co-pending PCT international application PCT/AU2019/051171.

The camera-based optical elastography device 500 is coupled wirelessly, such as using Wi-Fi or Bluetooth, to a microprocessor 516 in communication with a graphical interface 518, whereby a system 520 for evaluating a mechanical property of the sample material 512 is formed. The microprocessor 516 may be provided in the form of a computer such as a desktop computer, or in form of a mobile device, such as a tablet or a mobile phone. The microprocessor 516 is configured to receive, an electrical signal from the optical elastography device 500. The signal is indicative of information associated with the light detected by the CCD camera 514. The information can then be used by the microprocessor 516 and the graphical interface 518 and be converted into an image. The image is indicative of a distribution of stress and deformation across the sensing layer 508.

In another embodiment, the attachment is adapted for attaching to an optical probe of an OCT elastography device. In this embodiment, the sensing layer does not have a deformation-dependent optical property, but is transparent for the electromagnetic radiation and the OCT elastography device uses OCT to scan the depth of the sensing layer and obtain a depth profile of the sensing layer represented in an OCT image. The information obtained using OCT can then be used to characterise the mechanical property of the sample material, such as the elasticity of the sample material.

In again another embodiment, the attachment is adapted for attaching to an optical probe of an optical elastography device, which may be handheld and unlike OCT does not require scanning the entire thickness of the sensing layer for obtaining information relating to a mechanical property of the sample material. In this embodiment, the sensing layer is also transparent for the electromagnetic radiation and the mechanical property of the sample material can be derived based on the knowledge of the initial thickness of the sensing layer (before the axial load is applied) and based on the change in thickness of the layer as detected from the interface of signals reflected at bottom and top interfaces of the layer when the axial load is applied. The detected interference signals provide information about a change in the relative position of the interfaces between the sensing layer and the sample material and consequently about the change in thickness of the layer.

In another embodiment, the elastography device is not an optical elastography device but may be an MRI-based elastography device or an acoustic elastography device, such as an ultrasonic elastography device, comprising a probe having a transmission portion for transmission of acoustic waves.

Modifications and variations as would be apparent to a skilled addressee are determined to be within the scope of the present invention. For example, a skilled addressee will appreciate that in a variation of the described embodiment the sensing layer 108, 208 or 508 may comprise a compressible material such as a sponge-like material or another material that may have air pockets and a has a compressible volume.

## Claims

1. An attachment (100, 200) for an elastography and/or imaging device, the device having a transmission portion for transmission of electromagnetic radiation or acoustic waves towards a sample material, the attachment (100, 200) comprising:
a securing portion (104, 204) for securing the attachment (100, 200) to the device;
a ring-like portion (106, 206) coupled to the securing portion (104, 204);
a sensing portion coupled to the securing portion (104, 204), the sensing portion being adapted to receive a deformable sensing layer (108, 208) that is at least partially transmissive for the electromagnetic radiation or the acoustic waves;
a first strap or layer (118, 218) and a second strap or layer (120, 220), wherein, in use, the sensing layer (108, 208) is sandwiched between the first strap or layer (118, 218) and the second strap or layer (120, 220), and the first strap or layer (118, 218) and the second strap or layer (120, 220) are secured between the ring-like portion (106) and the securing portion (104);
wherein a cavity (109, 226) is defined between an outer portion of the first strap or layer (118, 218) and the second strap or layer (120, 220) at an edge-portion of the sensing layer (108, 208);
wherein the attachment (100, 200) is arranged such that, when attached to the device and the sensing layer (108, 208) is received at the sensing portion, the electromagnetic radiation is in use transmitted through the sensing layer (108, 208) or the acoustic waves are transmitted through the sensing layer (108, 208) towards the sample material, and when a load is applied to the sample material through the sensing layer (108, 208), the sensing layer (108, 208) deforms by expanding laterally relative to a longitudinal axis of the device, into the cavity (109, 226).

2. The attachment (100, 200) of claim 1, comprising the sensing layer (108, 208), wherein:
the sensing layer (108, 208) comprises a material that:
has a largely incompressible volume; and/or
is at least partially compressible.

3. The attachment (100, 200) of any one of the preceding claims wherein:
the attachment (100, 200) comprises the cavity (109, 226) into which the sensing layer (108, 208) can laterally expand when the load is applied to the sample material through the sensing layer (108, 208);
the attachment (100, 200) comprises a lubrication material at the sensing layer (108, 208) to reduce friction between the sensing layer (108, 208) and a component of the device with which the sensing layer (108, 208) is, in use, in contact, the lubrication material being arranged to penetrate into the cavity (109, 226) when the load is applied to the sample material through the sensing layer (108, 208); and/or
the attachment (100, 200) is arranged for attachment (100, 200) to a probe (102, 202, 503) of the device.

4. The attachment (100, 200) of any one of the preceding claims wherein the attachment (100, 200) comprises an end-portion at the sensing portion, the end-portion comprising an undulated edge having projections (113) projecting inwardly, the projections (113) being separated by recesses (115), wherein the end-portion is arranged such that a portion of the sensing layer (108, 208) can laterally expand into or through the recesses (115) between the projections (113) when an axial load is applied to the sample material through the sensing layer (108, 208).

5. The attachment (100, 200) of any one of claims 1 to **2** wherein:
the attachment (100, 200) further comprises a lubrication material at the sensing layer (108, 208) to reduce friction between the sensing layer (108, 208) and a component of the device with which the sensing layer (108, 208) is, in use, in contact; and
the attachment (100, 200) comprises an end-portion at the sensing portion, the end-portion comprising an undulated edge having projections (113) projecting inwardly, the projections (113) being separated by recesses (115) wherein the end-portion is arranged such that the lubrication material can penetrate into or through the recesses (115) between the projections (113) when an axial load is applied to the sample material through the sensing layer (108, 208).

6. The attachment (100, 200) of any one of the preceding claims, wherein:
the attachment (100, 200) is adapted for removably attaching to the device;
a cross-sectional shape of the attachment (100, 200) is approximately U-shaped when the sensing layer (108, 208) is received by the attachment (100, 200);
the attachment (100, 200) has a generally cylindrical shape; and/or
at least outer surface portions of the attachment (100, 200) are formed from a bio-compatible material.

7. The attachment (100, 200) of any one of the preceding claims, wherein:
the securing portion (104, 204) has at least one side portion arranged for engaging with a side portion of the device;
the sensing portion of the attachment (100, 200) is a bottom portion coupled to the at least one side portion;
the at least one side portion comprises a locking portion arranged to interlock with a corresponding locking portion of the side portion of the device; and/or
the at least one side portion of the attachment (100, 200) engages with the side portion of the device using:
a snap-fit;
a twist-lock or Luer-lock mechanism.

8. The attachment (100, 200) of any one of the preceding claims further comprising a protective sheath arranged to cover at least a portion of an exposed area of the attachment (100, 200), wherein:
the protective sheath extends along at least a portion of an exposed area of the device when the attachment (100, 200) is attached to the device; and/or
the protective sheath is a first protective sheath and is arranged to cover at least a portion of an exposed area of the attachment (100, 200), wherein the attachment (100, 200) further comprises a second protective sheath arranged to cover at least a portion of an exposed area of the device when the attachment (100, 200) is attached to the device.

9. The attachment (100, 200) of any one of the preceding claims wherein:
the device is:
an elastography device, the elastography device being:
an optical elastography device further comprising an optical imaging window (148, 242) at the sensing portion, the optical elastography device, with the attachment (100, 200), being arranged to direct the electromagnetic radiation from the elastography device through the imaging window (148, 242) and, when the sensing layer (108, 208) is received, subsequently through the sensing layer (108, 208), towards the sample material;
an optical coherence tomography elastography device;
an ultrasonic device; or
an MRI-based device;
the device comprises a probe (102, 202, 503) and the attachment (100, 200) is arranged for attachment (100, 200) to the probe (102, 202, 503);
the device is hand-held;
the attachment (100, 200) is disposable; and/or
the sensing layer (108, 208) comprises a silicone material.

10. An elastography and/or imaging system comprising:
an elastography and/or imaging device having a transmission portion for transmission of electromagnetic radiation or acoustic waves towards a sample material;
an attachment (100, 200) securable to the device, the attachment (100, 200) being provided in accordance with any one of the preceding claims; and
a deformable sensing layer (108, 208) coupled to the attachment (100, 200) at the sensing portion.

11. The system of any claim 10, wherein the device comprises an elongated probe (102, 202, 503) having an end portion for transmission of electromagnetic radiation or acoustic waves towards the sample material, and wherein the attachment (100, 200) is attached to the end portion of the elongated probe (102, 202, 503).

12. The system of any one of claims 10 to 11, wherein:
a sensing surface (121) of the sensing layer (108, 208) is adapted for positioning in direct or indirect contact with a surface area of the sample material;
the attachment (100, 200) comprises a flexible material that has a largely incompressible volume; and/or
the device is an optical elastography device and the sensing layer (108, 208) has a predetermined deformation-dependent optical property.

13. The system of claim 12, wherein the system is arranged such that a mechanical property of the sample material is measurable by detecting electromagnetic radiation or acoustic waves that transmitted through the sensing layer (108, 208) from the sample material in response to transmitting the electromagnetic radiation or acoustic waves through the sensing layer (108, 208) towards the sample material.

## Patentansprüche

1. Aufsatz (100, 200) für eine Elastographie- und/oder Bildgebungsvorrichtung,
wobei die Vorrichtung einen Übertragungsabschnitt zum Übertragen von elektromagnetischer Strahlung oder akustischen Wellen in Richtung eines Probenmaterials aufweist, wobei der Aufsatz (100, 200) umfasst:
einen Befestigungsabschnitt (104, 204) zum Befestigen des Aufsatzes (100, 200) an der Vorrichtung;
einen ringartigen Abschnitt (106, 206), der mit dem Befestigungsabschnitt (104, 204) gekoppelt ist;
einen Erfassungsabschnitt, der mit dem Befestigungsabschnitt (104, 204) gekoppelt ist, wobei der Erfassungsabschnitt dazu ausgelegt ist, eine verformbare Erfassungsschicht (108, 208) aufzunehmen, die zumindest teilweise für die elektromagnetische Strahlung oder die akustischen Wellen durchlässig ist;
einen ersten Streifen oder eine erste Schicht (118, 218) und einen zweiten Streifen oder eine zweite Schicht (120, 220),
wobei im Gebrauch die Erfassungsschicht (108, 208) zwischen dem ersten Streifen oder der ersten Schicht (118, 218) und dem zweiten Streifen oder der zweiten Schicht (120, 220) sandwichartig angeordnet ist und der erste Streifen oder die erste Schicht (118, 218) und der zweite Streifen oder die zweite Schicht (120, 220) zwischen dem ringartigen Abschnitt (106) und dem Befestigungsabschnitt (104) befestigt sind;
wobei zwischen einem äußeren Teil des ersten Streifens oder der ersten Schicht (118, 218) und dem zweiten Streifen oder der zweiten Schicht (120, 220) an einem Randabschnitt der Erfassungsschicht (108, 208) ein Hohlraum (109, 226) definiert ist;
wobei der Aufsatz (100, 200) derart angeordnet ist, dass, wenn er an der Vorrichtung angebracht ist und die Erfassungsschicht (108, 208) an dem Erfassungsabschnitt aufgenommen ist, die elektromagnetische Strahlung im Gebrauch durch die Erfassungsschicht (108, 208) übertragen wird oder die akustischen Wellen durch die Erfassungsschicht (108, 208) in Richtung des Probenmaterials übertragen werden und, wenn über die Erfassungsschicht (108, 208) eine Last auf das Probenmaterial aufgebracht wird, sich die Erfassungsschicht (108, 208) durch seitliches Ausdehnen relativ zu einer Längsachse der Vorrichtung in den Hohlraum (109, 226) hinein verformt.

2. Aufsatz (100, 200) nach Anspruch 1, umfassend die Erfassungsschicht (108, 208), wobei:
die Erfassungsschicht (108, 208) ein Material umfasst, das:
ein weitgehend inkompressibles Volumen aufweist; und/oder
zumindest teilweise kompressibel ist.

3. Aufsatz (100, 200) nach einem der vorhergehenden Ansprüche, wobei:
der Aufsatz (100, 200) den Hohlraum (109, 226) umfasst, in den sich die Erfassungsschicht (108, 208) seitlich ausdehnen kann, wenn die Last über die Erfassungsschicht (108, 208) auf das Probenmaterial aufgebracht wird;
der Aufsatz (100, 200) ein Schmiermaterial an der Erfassungsschicht (108, 208) umfasst, um Reibung zwischen der Erfassungsschicht (108, 208) und einer Komponente der Vorrichtung, mit der die Erfassungsschicht (108, 208) im Gebrauch in Kontakt steht, zu verringern, wobei das Schmiermaterial derart angeordnet ist, dass es in den Hohlraum (109, 226) eindringt, wenn die Last über die Erfassungsschicht (108, 208) auf das Probenmaterial aufgebracht wird; und/oder
der Aufsatz (100, 200) zur Befestigung (100, 200) an einer Sonde (102, 202, 503) der Vorrichtung eingerichtet ist.

4. Aufsatz (100, 200) nach einem der vorhergehenden Ansprüche, wobei der Aufsatz (100, 200) einen Endabschnitt an dem Erfassungsabschnitt umfasst, wobei der Endabschnitt einen wellenförmigen Rand mit nach innen vorspringenden Vorsprüngen (113) umfasst, wobei die Vorsprünge (113) durch Aussparungen (115) voneinander getrennt sind, wobei der Endabschnitt derart angeordnet ist, dass sich ein Teil der Erfassungsschicht (108, 208) seitlich in die Aussparungen (115) zwischen den Vorsprüngen (113) hinein oder durch diese hindurch ausdehnen kann, wenn eine axiale Last über die Erfassungsschicht (108, 208) auf das Probenmaterial aufgebracht wird.

5. Aufsatz (100, 200) nach einem der Ansprüche 1 bis 2, wobei:
der Aufsatz (100, 200) ferner ein Schmiermaterial an der Erfassungsschicht (108, 208) umfasst, um Reibung zwischen der Erfassungsschicht (108, 208) und einer Komponente der Vorrichtung, mit der die Erfassungsschicht (108, 208) im Gebrauch in Kontakt steht, zu verringern, und
der Aufsatz (100, 200) einen Endabschnitt an dem Erfassungsabschnitt umfasst, wobei der Endabschnitt einen wellenförmigen Rand mit nach innen vorspringenden Vorsprüngen (113) umfasst, wobei die Vorsprünge (113) durch Aussparungen (115) voneinander getrennt sind, wobei der Endabschnitt derart angeordnet ist, dass das Schmiermaterial in die Aussparungen (115) zwischen den Vorsprüngen (113) eindringen oder durch diese hindurchdringen kann, wenn eine axiale Last über die Erfassungsschicht (108, 208) auf das Probenmaterial aufgebracht wird.

6. Aufsatz (100, 200) nach einem der vorhergehenden Ansprüche, wobei:
der Aufsatz (100, 200) zur lösbaren Befestigung an der Vorrichtung ausgelegt ist;
eine Querschnittsform des Aufsatzes (100, 200) annähernd U-förmig ist, wenn die Erfassungsschicht (108, 208) von dem Aufsatz (100, 200) aufgenommen ist;
der Aufsatz (100, 200) eine im Allgemeinen zylindrische Form aufweist; und/oder
zumindest äußere Oberflächenabschnitte des Aufsatzes (100, 200) aus einem biokompatiblen Material gebildet sind.

7. Aufsatz (100, 200) nach einem der vorhergehenden Ansprüche, wobei:
der Befestigungsabschnitt (104, 204) mindestens einen Seitenabschnitt aufweist, der zum Eingriff mit einem Seitenabschnitt der Vorrichtung angeordnet ist;
der Erfassungsabschnitt des Aufsatzes (100, 200) ein Bodenabschnitt ist, der mit dem mindestens einen Seitenabschnitt gekoppelt ist;
der mindestens eine Seitenabschnitt einen Verriegelungsabschnitt umfasst, der zum Verriegelungseingriff mit einem entsprechenden Verriegelungsabschnitt des Seitenabschnitts der Vorrichtung angeordnet ist; und/oder
der mindestens eine Seitenabschnitt des Aufsatzes (100, 200) mit dem Seitenabschnitt der Vorrichtung in Eingriff steht unter Verwendung von:
einer Schnappverbindung;
einem Drehverschluss- oder Luer-Lock-Mechanismus.

8. Aufsatz (100, 200) nach einem der vorhergehenden Ansprüche, ferner umfassend eine Schutzhülle, die dazu angeordnet ist, zumindest einen Teil eines freiliegenden Bereichs des Aufsatzes (100, 200) abzudecken, wobei:
sich die Schutzhülle entlang zumindest eines Teils eines freiliegenden Bereichs der Vorrichtung erstreckt, wenn der Aufsatz (100, 200) an der Vorrichtung angebracht ist; und/oder
die Schutzhülle eine erste Schutzhülle ist und dazu angeordnet ist, zumindest einen Teil eines freiliegenden Bereichs des Aufsatzes (100, 200) abzudecken, wobei der Aufsatz (100, 200) ferner eine zweite Schutzhülle umfasst, die dazu angeordnet ist, zumindest einen Teil eines freiliegenden Bereichs der Vorrichtung abzudecken, wenn der Aufsatz (100, 200) an der Vorrichtung angebracht ist.

9. Aufsatz (100, 200) nach einem der vorhergehenden Ansprüche, wobei:
die Vorrichtung ist:
eine Elastographievorrichtung, wobei die Elastographievorrichtung ist:
eine optische Elastographievorrichtung, die ferner ein optisches Bildgebungsfenster (148, 242) an dem Erfassungsabschnitt umfasst, wobei die optische Elastographievorrichtung mit dem Aufsatz (100, 200) dazu angeordnet ist, die elektromagnetische Strahlung von der Elastographievorrichtung durch das Bildgebungsfenster (148, 242) und, wenn die Erfassungsschicht (108, 208) aufgenommen ist, anschließend durch die Erfassungsschicht (108, 208) in Richtung des Probenmaterials zu lenken;
eine Elastographievorrichtung für optische Kohärenztomographie;
eine Ultraschallvorrichtung; oder
eine MRT-basierte Vorrichtung;
die Vorrichtung eine Sonde (102, 202, 503) umfasst und der Aufsatz (100, 200) zur Befestigung (100, 200) an der Sonde (102, 202, 503) eingerichtet ist;
die Vorrichtung handgehalten ist;
der Aufsatz (100, 200) ein Einwegartikel ist; und/oder
die Erfassungsschicht (108, 208) ein Silikonmaterial umfasst.

10. Elastographie- und/oder Bildgebungssystem, umfassend:
eine Elastographie- und/oder Bildgebungsvorrichtung, die einen Übertragungsabschnitt zum Übertragen von elektromagnetischer Strahlung oder akustischen Wellen in Richtung eines Probenmaterials aufweist;
einen Aufsatz (100, 200), der an der Vorrichtung befestigbar ist, wobei der Aufsatz (100, 200) gemäß einem der vorhergehenden Ansprüche bereitgestellt ist; und
eine verformbare Erfassungsschicht (108, 208), die an dem Erfassungsabschnitt mit dem Aufsatz (100, 200) gekoppelt ist.

11. System nach Anspruch 10, wobei die Vorrichtung eine längliche Sonde (102, 202, 503) mit einem Endabschnitt zum Übertragen von elektromagnetischer Strahlung oder akustischen Wellen in Richtung des Probenmaterials umfasst und wobei der Aufsatz (100, 200) an dem Endabschnitt der länglichen Sonde (102, 202, 503) angebracht ist.

12. System nach einem der Ansprüche 10 bis 11, wobei:
eine Erfassungsoberfläche (121) der Erfassungsschicht (108, 208) dazu ausgelegt ist, in direkten oder indirekten Kontakt mit einem Oberflächenbereich des Probenmaterials gebracht zu werden;
der Aufsatz (100, 200) ein flexibles Material umfasst, das ein weitgehend inkompressibles Volumen aufweist; und/oder
die Vorrichtung eine optische Elastographievorrichtung ist und die Erfassungsschicht (108, 208) eine vorbestimmte verformungsabhängige optische Eigenschaft aufweist.

13. System nach Anspruch 12, wobei das System derart angeordnet ist, dass eine mechanische Eigenschaft des Probenmaterials durch Erfassen von elektromagnetischer Strahlung oder akustischen Wellen messbar ist, die durch die Erfassungsschicht (108, 208) von dem Probenmaterial übertragen wurden, als Reaktion darauf, dass die elektromagnetische Strahlung oder die akustischen Wellen durch die Erfassungsschicht (108, 208) in Richtung des Probenmaterials übertragen werden.

## Revendications

1. Accessoire (100, 200) pour un dispositif d'élastographie et/ou d'imagerie, le dispositif comportant une partie de transmission destinée à transmettre un rayonnement électromagnétique ou des ondes acoustiques vers un matériau échantillon, l'accessoire (100, 200) comprenant :
une partie de fixation (104, 204) destinée à fixer l'accessoire (100, 200) au dispositif ;
une partie en forme d'anneau (106, 206) couplée à la partie de fixation (104, 204) ;
une partie de détection couplée à la partie de fixation (104, 204), la partie de détection étant adaptée pour recevoir une couche de détection déformable (108, 208) qui est au moins partiellement transmissive pour le rayonnement électromagnétique ou les ondes acoustiques ;
une première bande ou couche (118, 218) et une seconde bande ou couche (120, 220),
dans lequel, lors de l'utilisation, la couche de détection (108, 208) est prise en sandwich entre la première bande ou couche (118, 218) et la seconde bande ou couche (120, 220), et la première bande ou couche (118, 218) et la seconde bande ou couche (120, 220) sont fixées entre la partie en forme d'anneau (106) et la partie de fixation (104) ;
dans lequel une cavité (109, 226) est définie entre une partie extérieure de la première bande ou couche (118, 218) et la seconde bande ou couche (120, 220) au niveau d'une partie de bord de la couche de détection (108, 208) ;
dans lequel l'accessoire (100, 200) est agencé de telle sorte que, lorsqu'il est fixé au dispositif et que la couche de détection (108, 208) est reçue au niveau de la partie de détection, le rayonnement électromagnétique est transmis, lors de l'utilisation, à travers la couche de détection (108, 208) ou les ondes acoustiques sont transmises à travers la couche de détection (108, 208) vers le matériau échantillon, et lorsqu'une charge est appliquée au matériau échantillon à travers la couche de détection (108, 208), la couche de détection (108, 208) se déforme en se dilatant latéralement par rapport à un axe longitudinal du dispositif, dans la cavité (109, 226).

2. Accessoire (100, 200) selon la revendication 1, comprenant la couche de détection (108, 208), dans lequel :
la couche de détection (108, 208) comprend un matériau qui :
présente un volume largement incompressible ; et/ou
est au moins partiellement compressible.

3. Accessoire (100, 200) selon l'une quelconque des revendications précédentes, dans lequel :
l'accessoire (100, 200) comprend la cavité (109, 226) dans laquelle la couche de détection (108, 208) peut se dilater latéralement lorsque la charge est appliquée au matériau échantillon à travers la couche de détection (108, 208) ;
l'accessoire (100, 200) comprend un matériau lubrifiant au niveau de la couche de détection (108, 208) pour réduire le frottement entre la couche de détection (108, 208) et un composant du dispositif avec lequel la couche de détection (108, 208) est, lors de l'utilisation, en contact, le matériau lubrifiant étant agencé pour pénétrer dans la cavité (109, 226) lorsque la charge est appliquée au matériau échantillon à travers la couche de détection (108, 208) ; et/ou
l'accessoire (100, 200) est agencé pour être fixé (100, 200) à une sonde (102, 202, 503) du dispositif.

4. Accessoire (100, 200) selon l'une quelconque des revendications précédentes, dans lequel l'accessoire (100, 200) comprend une partie d'extrémité au niveau de la partie de détection, la partie d'extrémité comprenant un bord ondulé présentant des saillies (113) faisant saillie vers l'intérieur, les saillies (113) étant séparées par des évidements (115), la partie d'extrémité étant agencée de telle sorte qu'une partie de la couche de détection (108, 208) puisse se dilater latéralement dans les évidements (115) entre les saillies (113) ou à travers ceux-ci lorsqu'une charge axiale est appliquée au matériau échantillon à travers la couche de détection (108, 208).

5. Accessoire (100, 200) selon l'une quelconque des revendications 1 à 2, dans lequel :
l'accessoire (100, 200) comprend en outre un matériau lubrifiant au niveau de la couche de détection (108, 208) pour réduire le frottement entre la couche de détection (108, 208) et un composant du dispositif avec lequel la couche de détection (108, 208) est, lors de l'utilisation, en contact, et
l'accessoire (100, 200) comprend une partie d'extrémité au niveau de la partie de détection, la partie d'extrémité comprenant un bord ondulé présentant des saillies (113) faisant saillie vers l'intérieur, les saillies (113) étant séparées par des évidements (115), la partie d'extrémité étant agencée de telle sorte que le matériau lubrifiant puisse pénétrer dans les évidements (115) entre les saillies (113) ou à travers ceux-ci lorsqu'une charge axiale est appliquée au matériau échantillon à travers la couche de détection (108, 208).

6. Accessoire (100, 200) selon l'une quelconque des revendications précédentes, dans lequel :
l'accessoire (100, 200) est adapté pour être fixé de manière amovible au dispositif ;
une forme en coupe transversale de l'accessoire (100, 200) est approximativement en forme de U lorsque la couche de détection (108, 208) est reçue par l'accessoire (100, 200) ;
l'accessoire (100, 200) présente une forme généralement cylindrique ; et/ou
au moins des parties de surface extérieure de l'accessoire (100, 200) sont formées d'un matériau biocompatible.

7. Accessoire (100, 200) selon l'une quelconque des revendications précédentes, dans lequel :
la partie de fixation (104, 204) comporte au moins une partie latérale agencée pour venir en prise avec une partie latérale du dispositif ;
la partie de détection de l'accessoire (100, 200) est une partie inférieure couplée à l'au moins une partie latérale ;
l'au moins une partie latérale comprend une partie de verrouillage agencée pour venir en prise par verrouillage avec une partie de verrouillage correspondante de la partie latérale du dispositif ; et/ou
l'au moins une partie latérale de l'accessoire (100, 200) vient en prise avec la partie latérale du dispositif en utilisant :
un ajustement par encliquetage ;
un mécanisme de verrouillage par rotation ou de type Luer-lock.

8. Accessoire (100, 200) selon l'une quelconque des revendications précédentes, comprenant en outre une gaine de protection agencée pour couvrir au moins une partie d'une zone exposée de l'accessoire (100, 200), dans lequel :
la gaine de protection s'étend le long d'au moins une partie d'une zone exposée du dispositif lorsque l'accessoire (100, 200) est fixé au dispositif ; et/ou
la gaine de protection est une première gaine de protection et est agencée pour couvrir au moins une partie d'une zone exposée de l'accessoire (100, 200), l'accessoire (100, 200) comprenant en outre une seconde gaine de protection agencée pour couvrir au moins une partie d'une zone exposée du dispositif lorsque l'accessoire (100, 200) est fixé au dispositif.

9. Accessoire (100, 200) selon l'une quelconque des revendications précédentes, dans lequel :
le dispositif est :
un dispositif d'élastographie, le dispositif d'élastographie étant : un dispositif d'élastographie optique comprenant en outre une fenêtre d'imagerie optique (148, 242) au niveau de la partie de détection, le dispositif d'élastographie optique, avec l'accessoire (100, 200), étant agencé pour diriger le rayonnement électromagnétique provenant du dispositif d'élastographie à travers la fenêtre d'imagerie (148, 242) et, lorsque la couche de détection (108, 208) est reçue, ensuite à travers la couche de détection (108, 208), vers le matériau échantillon ;
un dispositif d'élastographie par tomographie par cohérence optique ;
un dispositif à ultrasons ; ou
un disositif basé sur l'IRM ;
le dispositif comprend une sonde (102, 202, 503) et l'accessoire (100, 200) est agencé pour être fixé (100, 200) à la sonde (102, 202, 503) ;
le dispositif est portatif ;
l'accessoire (100, 200) est jetable ; et/ou
la couche de détection (108, 208) comprend un matériau en silicone.

10. Système d'élastographie et/ou d'imagerie comprenant :
un dispositif d'élastographie et/ou d'imagerie comportant une partie de transmission destinée à transmettre un rayonnement électromagnétique ou des ondes acoustiques vers un matériau échantillon ;
un accessoire (100, 200) pouvant être fixé au dispositif, l'accessoire (100, 200) étant conforme à l'une quelconque des revendications précédentes ; et
une couche de détection déformable (108, 208) couplée à l'accessoire (100, 200) au niveau de la partie de détection.

11. Système selon la revendication 10, dans lequel le dispositif comprend une sonde allongée (102, 202, 503) comportant une partie d'extrémité destinée à transmettre un rayonnement électromagnétique ou des ondes acoustiques vers le matériau échantillon, et dans lequel l'accessoire (100, 200) est fixé à la partie d'extrémité de la sonde allongée (102, 202, 503).

12. Système selon l'une quelconque des revendications 10 à 11, dans lequel :
une surface de détection (121) de la couche de détection (108, 208) est adaptée pour être positionnée en contact direct ou indirect avec une zone de surface du matériau échantillon ;
l'accessoire (100, 200) comprend un matériau flexible qui présente un volume largement incompressible ; et/ou
le dispositif est un dispositif d'élastographie optique et la couche de détection (108, 208) présente une propriété optique prédéterminée dépendant de la déformation.

13. Système selon la revendication 12, dans lequel le système est agencé de telle
sorte qu'une propriété mécanique du matériau échantillon soit mesurable par détection d'un rayonnement électromagnétique ou d'ondes acoustiques transmis à travers la couche de détection (108, 208) depuis le matériau échantillon en réponse à la transmission du rayonnement électromagnétique ou des ondes acoustiques à travers la couche de détection (108, 208) vers le matériau échantillon.
